## Europäisches Patentamt
## European Patent Office
## Office européen des brevets

Veröffentlichungsnummer: **0 275 400**
**A1**

# EUROPÄISCHE PATENTANMELDUNG

Anmeldenummer: **87117055.1**

Anmeldetag: **19.11.87**

Int. Cl.4 **C07C 37/60 , C07C 39 08**

Priorität: **18.12.86 DE 3643206**

Veröffentlichungstag der Anmeldung:
**27.07.88 Patentblatt 88/30**

Benannte Vertragsstaaten:
**AT BE CH DE ES FR GB IT LI LU NL SE**

Anmelder: **Degussa Aktiengesellschaft**
**Weissfrauenstrasse 9**
**D-6000 Frankfurt am Main 1(DE)**

Erfinder: **Drauz, Karl-Heinz, Dr.**
**Zur Marienruhe 1**
**D-6463 Freigericht 1(DE)**
Erfinder: **Kleemann, Axel, Dr.**
**Bornweg 36**
**D-6052 Mühlheim/Main(DE)**
Erfinder: **Prescher, Günter, Dr.**
**Liesingstrasse 2**
**D-6450 Hanau 9(DE)**
Erfinder: **Ritter, Gebhard, Dr.**
**Fürstenbergstrasse 2**
**D-6450 Hanau 9(DE)**

## Verfahren zur Herstellung von substituierten Dihydroxybenzolen.

Die Kernhydroxylierung von substituierten Phenolen ließ sich durch Verwenden von wäßrigem Wasserstoffperoxyd in Gegenwart von Schwefeldioxid oder Selendioxid als Katalysator in technisch einfacher Weise bei sehr guten Raum-Zeitausbeuten und guten Produktausbeuten durchführen.

EP 0 275 400 A1

## Verfahren zur Herstellung von substituierten Dihydroxybenzolen

Die Erfindung betrifft die Herstellung von substituierten Dihydroxybenzolen durch Hydroxylieren von substituierten Phenolen mit wäßrigem Wasserstoffperoxid.

Dihydroxybenzole werden bei der Herstellung von Farbstoffen, in der Kunststoffindustrie sowie in der Foto-und Pflanzenschutzmittel-Industrie eingesetzt.

Es ist nach dem Verfahren der DE-PS 20 64 497 bekannt, die Kernhydroxylierung aromatischer Verbindungen, vor allem von Phenol, mit Wasserstoffperoxid in Gegenwart einer starken Säure durchzuführen. Das Reaktionsmedium soll dabei zu Anfang nicht mehr als 20 Gew.-% Wasser, bevorzugt unter 10 Gew.-% Wasser, enthalten.

Der pH-Wert der starken Säuren wird unter -0,1, vorzugsweise unter -1, angegeben. Schwefelsäure und Perchlorsäure scheinen bevorzugt zu sein.

Dieses Verfahren wird aber von der Anmelderin der DE-PS 20 64 497 selbst in ihrer späteren Patentschrift DE-PS 26 58 545 negativ beurteilt. So sollen zwar die Ausbeuten an Hydroxylierungsprodukten bei gleichzeitiger Verwendung der starken Säuren und von Komplexbildnern für Metalle wie Pyrophosphorsäure "ausgezeichnet" sein, der Umwandlungsgrad der aromatischen Verbindung aber unter 30 % liegen. In der Praxis würden sogar Werte von 4 bis 10 % Umwandlungsgrad nicht überschritten.

Dies bedeute eine Begrenzung der Produktivität der Apparatur und die Rückführung eines bedeutenden Volumens an Ausgangsstoff.

Daher wäre eine möglichst hohe Reaktionsgeschwindigkeit wichtig. Diese hinge - bei gegebener Temperatur und Wassermenge - von Art und Menge der eingesetzten Säure ab. Unabhängig von der Art der Säure wäre es aber wünschenswert, die Reaktionsgeschwindigkeit zu erhöhen, und zwar ohne Erhöhung der Säuremenge, da letztere durch Auswaschen verloren ginge und außerdem auch die Korrosion durch die starke Säure nicht außer acht gelassen werden könne.

So wird in der DE-PS 26 58 545 als Verbesserung des obengenannten Verfahrens noch neben den dort genannten Katalysatoren und Stabilisatoren die zusätzliche Verwendung von aromatischen Aldehyden, wie Benzaldehyd, vorgeschlagen. Die Hydroxylierung von Phenol und substituierten Phenolen mit Wasserstoffperoxid findet also in Gegenwart starker Säuren, von Metallkomplexbildnern und aromatischen Aldehyden statt.

Anstelle von zwei, die Reaktion beeinflussenden Komponenten, werden nun also drei eingesetzt. Diese Komponenten müssen nicht nur aus dem Reaktionsgemisch abgetrennt werden und sind - als nicht rückgewinnbar - verloren, sondern die Komponente "aromatischer Aldehyd" unterliegt auch einer Oxydation mit Wasserstoffperoxid, was die Gefahr einer Verunreinigung des Endproduktes einschließt.

Höhere Ausbeuten von etwa 70 - 76 % - und in zwei Fällen über 80 % - werden aber auch hier nur dann erhalten, wenn Phenol Wasserstoffperoxidverhältnisse von 20 : 1 und Wasserstoffperoxid von etwa 85 % eingesetzt werden. In diesen Fällen muß aber, wie in der DE-PS 20 64 497, "ein bedeutendes Volumen an Ausgangsstoff rückgeführt" werden, was entsprechende technische Anlagen benötigt. Auch die Reaktoren selbst müssen dementsprechend groß ausgelegt werden.

Da beim Verfahren der DE-PS 20 64 497 die Ausbeute an Brenzkatechin und Hydrochinon bei Verringerung des Verhältnisses von Phenol zu Wasserstoffperoxid erheblich sinkt - bei einem Verhältnis von 10 : 1 liegt sie bei 60 %, bei einem solchen von 5 :1 bei 47 %, siehe Beispiel 7 - - scheint die Anwesenheit eines großen Überschusses an Phenol bei der Durchführung der Hydroxylierung mit starken Säuren eine Notwendigkeit zu sein, ganz abgesehen davon, daß die in der DE-PS 20 64 497 genannten starken Säuren, wie Schwefelsäure, Perchlorsäure als Katalysatoren nur eine unzureichende Wirkung hatten. Bei substituierten Phenolen waren die Ausbeuten noch - schlechter.

Man hatte deshalb schon angenommen, durch Verwendung von wasserfreien Lösungen von Wasserstoffperoxid die katalytische Wirkung der starken Säuren zu verbessern, siehe DE-PS 24 10 742 und DE-OS 24 10 758; jedoch wurde auch hier die Anwesenheit von Phosphorverbindungen als Komplexbildnern für wesentlich gehalten. Außerdem wurde darauf hingewiesen, daß die Reaktion am - schnellsten bei hoher Säurekonzentrationen verlaufe. Die Frage der Korrosion durch starke Säuren war also auch hier nicht gelöst.

Einen wesentlichen Fortschritt gegenüber den genannten Verfahren brachten die Verfahren der DE-PS 33 08 737, DE-PS 33 08 769, DE-PS 33 08 763 und DE-PS 33 08 726, bei denen die Hydroxylierung von Phenol oder seinen Derivaten durch organische Lösungen von Wasserstoffperoxid in Gegenwart von Schwefeldioxid oder Selendioxid durchgeführt wurde.

Bei diesen Verfahren traten die störenden Folgen der starken Säuren, wie Korrosion, nicht ein, noch war es nötig, zur Erhöhung der Aktivität zusätzliche Verbindungen, wie Aldehyde, oder auch Komplexbildner, wie Phosporderivate, einzusetzen.

Obwohl die Katalysatoren in sehr geringen Mengen eingesetzt wurden, war die Reaktionsgeschwindigkeit groß: es wurden sehr günstige Raum-Zeitausbeuten und sehr gute Ausbeuten erhalten. Durch ihre sehr geringe Menge beanspruchen Schwefeldioxid und Selendioxid auch keine speziellen Abtrennmethoden.

Nach dem Stand der Technik schien zunächst einmal nur die Verwendung von wasserfreien Lösungen von Wasserstoffperoxid zu besseren Umsätzen und Ausbeuten zu führen. Bei Verwendung von starken Säuren als Katalysator bestand aber nach wie vor die Schwierigkeit von deren Abtrennung sowie das Auftreten von Korrosion.

Hier brachte der Einsatz von Schwefeldioxid oder Selendioxid einen wesentlichen Fortschritt. Nur beanspruchten auch diese Verfahren wasserfreie Lösungen von Wasserstoffperoxid, die nicht mehr als 1 Gew.-%, bevorzugt unter 0,5 Gew.-% Wasser, enthalten sollten. Diese Arbeitsweise erfordert auch zusätzliche Anlagen zur Abtrennung und Rückführung des organischen Lösungsmittels.

Aufgabe der vorliegenden Erfindung ist es nun, die Hydroxylierung von substituierten Phenolen mit Schwefeldioxid oder Selendioxid in technisch einfacherer Form durchzuführen.

Es wurde nun gefunden, daß sich diese Aufgabe lösen läßt, wenn man die Hydroxylierung von substituierten Phenolen in Gegenwart von Schwefeldioxid oder Selendioxid als Katalysator mit wäßrigen Wasserstoffperoxidlösungen durchführt. Das Verhältnis der substituierten Phenole zu Wasserstoffperoxid liegt bei 5 bis 25 : 1, bevorzugt 10 bis 16 : 1 (in Molen).

Wasserstoffperoxid wird in handelsüblichen Lösungen in den entsprechenden Konzentrationen eingesetzt; die Umsetzung wird mit 30 - 85 gew.-%igen Lösungen, bevorzugt 70 - 85 gew.-%igen Lösungen, durchgeführt. Das eingesetzte Schwefeldioxid ist 99,75 gew.-%ig.

Die Katalysatoren Schwefeldioxid oder Selendioxid werden in Mengen von 0,0001 bis 0,04 Mol, bezogen auf 1 Mol Wasserstoffperoxid, verwendet.

Schwefeldioxid wird flüssig oder gasförmig aus handelsüblichen Stahlflaschen oder als Lösungen in z.B. Alkylestern, Alkanen wie z.B. Cyclohexan, oder den substituierten Phenolen eingesetzt.

$SeO_2$ wird in p.a. Form eingesetzt. Die Umsetzungen werden bei Temperaturen von 20 - 160 °C durchgeführt. Der Druck ist nicht entscheidend; im allgemeinen wird bei Normaldruck gearbeitet.

Als substituierte Phenole kommen z.B. infrage: Alkylabkömmlinge von Phenol, wie die Kresole, Äthyloder Butylphenole und von letzteren besonders o-und -p-tert.-Butylphenol. vor allem p-tert.-Butylphenol. In p-Stellung substituierte Phenole waren allgemein recht geeignet für das erfindungsgemäße Verfahren. Außerdem erwiesen sich auch Alkoxyphenole, wie z.B. Anisol oder Arylphenole, wie z.B. 4-Hydroxydiphenyl, als günstige Ausgangsprodukte, ebenso halogenierte Phenole, wie z.B. o-und p-Chlorphenol und Phenyläthylbzw. Phenylisopropyläther.

Bevorzugt waren p-tert.-Butylphenol, Anisol und p-Kresol. Die einzusetzenden Stoffe können sowohl als Schmelzen oder in geeigneten inerten organischen Lösungsmitteln, wie aromatischen Kohlenwasserstoffen, z.B. Benzol Toluol, cycloaliphatischen Kohlenwasserstoffen, wie z.B. Cyclohexan, aromatischen und aliphatischen Estern, z.B. Propylacetat, oder chlorierten Kohlenwasserstoffen, wie z.B. Chloroform, Tetrachlorkohlenstoff verwendet werden.

Das Verfahren kann sowohl diskontinuierlich als auch kontinuierlich durchgeführt werden. Das bei dem erfindungsgemäßen Verfahren rezyklierte substituierte Phenol bedarf keiner besonderen Behandlung.

Der technische Fortschritt des erfindungsgemäßen Verfahrens liegt einmal in seiner einfachen technischen Durchführung: anstelle von organischen Wasserstoffperoxidlösungen, bei denen das organische Lösungsmittel abgetrennt werden muß, kann direkt wäßriges Wasserstoffperoxid eingesetzt werden. Ferner werden durch Verwendung von Schwefeldioxid oder Selendioxid in äußerst geringen Mengen die Schwierigkeiten vermieden, die durch Verwendung von starken Säuren auftreten, wie aufwendiges Abtrennen dieser Katalysatoren und Auftreten von Korrosionen. Auch fällt das Aufsalzen der Abwässer durch Neutralisatiom der Säuren fort.

Es kommt hinzu, daß Aktivatoren für eine ausreichende Reaktionsgeschwindigkeit bei Schwefeldioxid und Selendioxid nicht nötig sind, ebenso nicht der Zusatz von Stabilisatoren. Damit werden zusätzliche technische Einrichtungen und bei der Sauerstoffempfindlichkeit gerade der wirkungsvollen Aktivatoren auch die Verschmutzung des Endproduktes durch Oxydationsprodukte der Aktivatoren vermieden.

Ferner führt die hohe Reaktionsgeschwindigkeit zu einer erhöhten Raum-Zeitausbeute.

Zu der vereinfachten technischen Durchführung kommt aber hinzu, daß der Umsatz an Wasserstoffperoxid hoch und die Raum-Zeitausbeute höher als mit organischen Wasserstoffperoxidlösungen ist. Außerdem ist die Ausbeute an den Hydroxybenzolen mindestens so hoch wie bei den Verfahren des Standes der Technik.

Die Erfindung wird durch die nachstehenden Beispiele weiter erläutert. Die Ergebnisse wurden durch Gas-Flüssigkeitschromatografie (GLC) bestimmt. Es wurde unter Stickstoff gearbeitet, obwohl auch bei Anwesenheit von Luft keine Änderungen an den Ergebnissen beobachtet wurden.

Beispiel 1 75 g (0,5 mol) p-tert.-Butylphenol werden auf 102 °C erwärmt, und man löst darin 100 mg (1,56 mmol) gasförmiges Schwefeldioxid. Zu dieser Mischung gibt man dann 1,51 g (0,031 mol) 70 gew.-%iges Wasserstoffperoxyd hinzu. Die Temperatur in der Reaktionslösung erhöht sich danach auf 128 °C.
Nach Abklingen der Exotherme wird nach 10 Minuten ein Wasserstoffperoxid-Umsatz von größer 99 % bestimmt. Das Reaktionsgemisch enthält dann 4,2 g 4-tert.-Butylbrenzkatechin, was einer Ausbeute von 81.5 %, bezogen auf das eingesetzte Wasserstoffperoxyd, entspricht.

Beispiel 2

75 g (0,5 mol) p-tert.-Butylphenol werden auf 102 °C erwärmt, und man versetzt diese mit 50 mg (0,78 mmol) Schwefeldioxid. Zu dieser Mischung gibt man dann 0,8 g (20 mmol) 85 gew.-%iges Wasserstoffperoxyd hinzu. Die Temperatur in der Reaktionslösung erhöht sich danach auf 118 °C.
Nach Abklingen der Exotherme wird nach 10 Minuten ein Wasserstoffperoxyd-Umsatz von größer 99 % bestimmt. Das Reaktionsgemisch enthält dann 2,77 g 4-tert.-Butylbrenzkatechin, was einer Ausbeute von 83,3 %, bezogen auf das eingesetzte Wasserstoffperoxyd, entspricht.

Beispiel 3

75 g (0,5 mol) p-tert.-Butylphenol werden auf 103 °C erwärmt. Zu der gerührten Schmelze gibt man 0,4 g einer 60 gew.-%igen Lösung von Schwefeldioxid in Essigsäuren-propylester und anschließend 1,51 g (0,031 mol) 70 gew.-%iges Wasserstoffperoxyd. Die Temperatur in der Reaktionsmischung erhöht sich danach auf 131 °C.
Nach Abklingen der Exotherme wird nach 10 Minuten ein Wasserstoffperoxid-Umsatz von 99 % bestimmt. Das Reaktionsgemisch enthält dann 4,11 g 4-tert.-Butylbrenzkatechin, was einer Ausbeure von 80 %, bezogen auf das eingesetzte Wasserstoffperoxyd, entspricht.

Beispiel 4

54 g (0,5 mol) Anisol werden auf 120 °C erwärmt, und man versetzt diese Lösung mit 50 mg (0,78 mmol) Schwefeldioxid. Zu dieser Mischung gibt man dann 1,18 g (0,029 mol) 84,4 gew.-%iges Wasserstoffperoxyd hinzu. Die Temperatur in der Reaktionslösung erhöht sich danach auf 138 °C. Nach Abklingen der Exotherme wird nach 10 Minuten ein Wasserstoffperoxyd-Umsatz von 98 % bestimmt. Das Reaktionsgemisch enthält dann 1,88 g (0,015 mol) Brenzkatechinmonomethylether und 0,76 g (6 mmol) Hydrochinonmonomethylether, was einer Gesamtausbeute von 73,3 %, bezogen auf das eingesetzte Wasserstoffperoxyd, entspricht.

Beispiel 5

54 g (0,5 mol) p-Kresol werden auf 105 °C erwärmt, und man versetzt diese Lösung mit 50 mg (0,78 mmol) Schwefeldioxid. Zu dieser Mischung gibt man dann 1,51 g (0,031 mol) 70 gew.-%iges Wasserstoffperoxyd hinzu. Die Temperatur in der Reaktionslösung erhöht sich danach auf 135 °C.
Nach Abklingen der Exotherme wird nach 10 Minuten ein Wasserstoffperoxyd-Umsatz von 99 % bestimmt. Das Reaktionsgemisch enthält dann 2,77 g 4-Methylbrenzkatechin und 0,31 g 4-Methylresorcin, was einer Gesamtausbeute an Dihydroxybenzolen von 80,1 %, bezogen auf das eingesetzte Wasserstoffperoxyd, entspricht.

Ansprüche

1.) Verfahren zur Herstellung von substituierten Dihydroxybenzolen durch Kernhydroxylierung der entsprechenden substituierten Phenole mit Wasserstoffperoxid in Gegenwart von Schwefeldioxid oder Selendioxid als Katalysator, dadurch gekennzeichnet, daß man die Umsetzung mit wäßrigem Wasserstoffperoxid durchführt.

2. ) Verfahren nach Anspruch 1, dadurch gekennzeichnet, daß man die Umsetzung mit 70 - 85 gew.-%igem wäßrigem Wasserstoffperoxid durchführt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| E | EP-A-0 230 625  (DEGUSSA)<br>* Ansprüche 1-4 *<br>--- | 1,2 | C 07 C  37/60<br>C 07 C  39/08 |
| A | DE-A-2 348 957  (RHONE-POULENC)<br>* Anspruch 1; Seite 3, Zeile 26 - Seite 4, Zeile 2 *<br>--- | 1,2 | |
| A | US-A-3 953 530  (DULOG et al.)<br>* Ansprüche 1,2 *<br>----- | 1 | |

| RECHERCHIERTE SACHGEBIETE (Int. Cl.4) |
|---|
| C 07 C  37/00<br>C 07 C  39/00 |

Der vorliegende Recherchenbericht wurde für alle Patentansprüche erstellt

| Recherchenort | Abschlußdatum der Recherche | Prüfer |
|---|---|---|
| DEN HAAG | 25-03-1988 | KLAG M.J. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)